# EUROPEAN PATENT APPLICATION

(11) **EP 0 632 215 A1**
(43) Date of publication of application: **04.01.1995**
(21) Application number: 93113284.9
(22) Date of filing: 19.08.1993
(51) Int. Cl.: F16H 21/40, A61B 17/14

(54) **Crank gearing for converting rotary motion into oscillating motion**

(30) Priority: 30.06.1993 JP 160654/93
(71) Applicant: OSADA RESEARCH INSTITUTE, LTD., Tokyo (JP)
(72) Inventor: Seto, Norio c/o Osada Research Institute, Ltd., Shinagawa-Ku Tokyo (JP); Iizuka, Takeshi c/o Osada Research Institute,Ltd., Shinagawa-Ku Tokyo (JP)
(74) Representative: Grünecker, Kinkeldey, Stockmair & Schwanhäusser Anwaltssozietät

(57) **Abstract**

A power transmission device is described, which device converts rotational movement of a rotation shaft (3) into reciprocal turning movement of a turning shaft (15) extending parallel to the rotation shaft, and which is simple in its mechanical construction and small in size of its body case. Rotational movement of a first rotation shaft (3) is converted into reciprocal turning movement of a turning shaft (15). Rotation of the first rotation shaft is transmitted through a second rotation shaft (3a) rotatable with an eccentricity to the first rotation shaft and a bearing (4) mounted on an end of the second rotation shaft to a rocking member which is turnable in response to only a circular displacement of the second rotation shaft. The swinging center of the rocking member (5) is fixed with a bolt (5c) or the like to the turning shaft extending parallel to the first rotation shaft. The turning shaft reciprocally turns as the first rotation shaft rotates.

## Description

### BACK GROUND OF THE INVENTION

The present invention relates to a power transmission device and more particularly to a device for converting rotational movement of a rotation shaft into reciprocal turning movement of a turning shaft extending parallel to the rotation shaft.

In dental treatment, tooth cutting is carried out by using cutting tools to be driven by a micromotor rotating at high speed. In recent years, with the progress of subdivision of medical areas and specialization of medical technology, such specialists that are engaged, for example, in medical treatment of hands or feet only have already appeared. In medical treatment of hands or feet, it is also necessary to carry out operations such as bone amputation or cutting, but no cutting device which is suitable for use in medical treatment of hands or feet, has been developed. Every specialist feels inconvenienced not having it.

In view of the foregoing, the present applicant previously proposed a cutting device which is suitable for amputating and/or cutting fine bones of hands or feet of human or animals. The proposed cutting device is not limited to the above-mentioned application and can be also used for amputating and/or cutting bones of human, animals or other kinds of material, e.g., wood and so on.

In a power transmission device proposed by the present applicant, a first rotation shaft connected to a rotation shaft to be driven by, for example, a micromotor known for use in dental treatment devices. The first rotation shaft is preferably connected to a driving portion of a motor-driven hand-piece for a dental treatment device, which is available on the market.

Rotational movement of the first rotation shaft is transmitted through an inclined rotation transmitting mechanism to a second rotation shaft eccentrically positioned in relation to the first rotation shaft. The second rotation shaft is provided at its front end with a bearing which rotates with an eccentricity to the second rotation shaft to swing a rocking member which has, at its side opposite to the bearing, a concave portion having the same width in circular direction as the diameter of the bearing and larger width in radial direction than the maximum eccentricity of the bearing. The bearing is fitted in the concave portion of the rocking member.

Accordingly, when the second rotation shaft rotates, the rocking member reciprocally revolves round its axis. The rocking member is supported by supporting member, for example, as fitted in a hollow hole thereof. The supporting member is rotatably mounted in a fixing member by means of a bearing.

The supporting member has a hollow hole with a thread for threadedly engaging a fixing bolt. A sawing plate can be secured at its end between the bearing fixing member and a washer by tightening the bolt. While the rotation shaft rotates, the sawing plate together with the supporting member reciprocally revolve round the axis of supporting member.

The above-mentioned power transmission device, whereby rotational movement of the first rotation shaft is transmitted to a coaxially rotatable supporting member to cause coaxial reciprocal revolution thereof, has to utilize an inclined rotation transmission mechanism to make the supporting member aligned with the rotation shaft, that not only complicates the construction of the device, but also requires the large-sized body case for accommodating the mechanism. This restricts the possibility of reducing the dimension of the device.

### SUMMARY OF THE INVENTION

It is an object of the present invention to provide power transmission device which is constructed to convert rotational movement of the rotation shaft into reciprocal turning movement of a shaft extending parallel to the rotation shaft, thereby its mechanical construction is simplified with a reduced size of a body case for accommodating therein the turning shaft.

It is another object of the present invention to provide a power transmission device for converting rotational movement of a rotation shaft into reciprocal turning movement, comprising a first rotation shaft, a second rotation shaft rotatable with an eccentricity to the first rotation shaft, a bearing mounted on an end of the second rotation shaft, a rocking member swingable in response to only a circular displacement of the second rotation shaft round its revolving axis, and a turning shaft extending parallel to the first rotation shaft and fixing a swinging axis of the rocking member, characterized in that the device converts rotation of the first rotation shaft into reciprocal turning movement of the turning shaft.

### BRIEF DESCRIPTION OF DRAWINGS

Figs. 1(a) and 1(b) are an essential construction views for explaining an embodiment of a power transmission device previously proposed by the present applicant.

Figs. 2(a) and (2b) are an essential construction views for explaining an embodiment of a power transmission device according to the present invention.

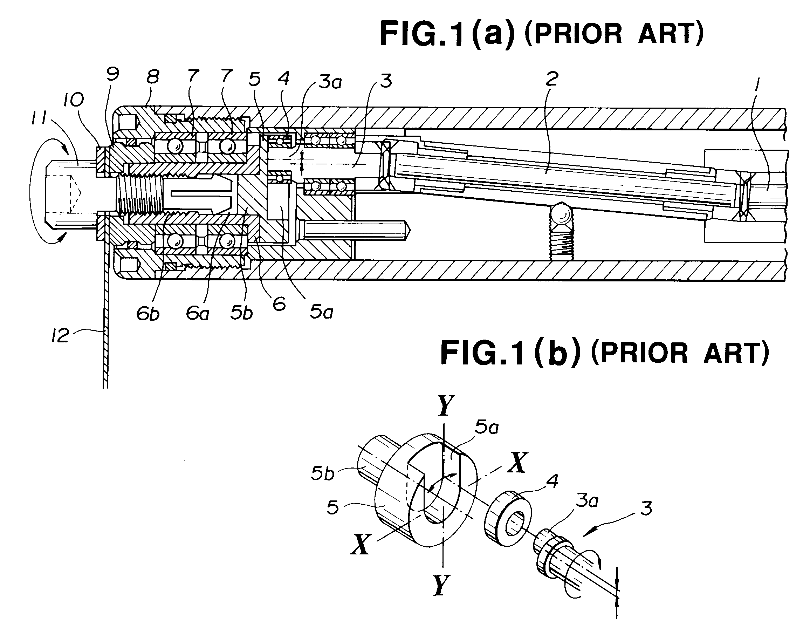

Figs. 1(a) and 1(b) are a sectional views for explaining an example of a power transmission device proposed by the present applicant. In Figs. 1(a) and 1(b), numeral 1 denotes a first rotation shaft connected to a second rotation shaft to be driven by, for example, a micromotor known for use in dental treatment devices. The first rotation shaft is preferably connected to a driving portion of a motor-driven hand-piece for a dental treatment device, which is available on the market.

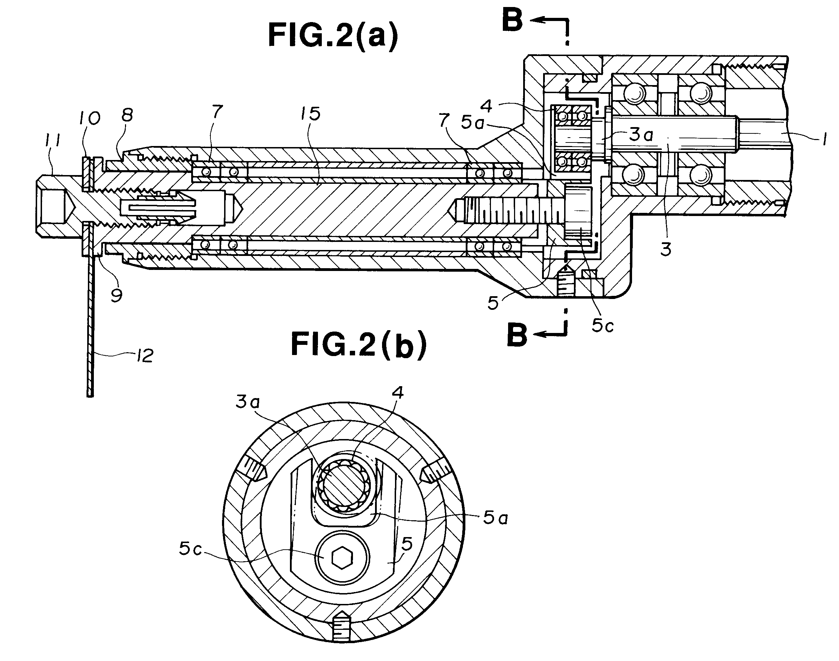

Rotational movement of the first rotation shaft 1 is transmitted through an inclined rotation transmitting mechanism 2 to a second rotation shaft 3 eccentrically positioned in relation to the first rotation shaft 1. The second rotation shaft 3 is provided at its front end with a bearing 3a which rotates with an eccentricity to the second rotation shaft 3 as shown in Fig. 2(b) to swing a rocking member 5 which has, at its side opposite to the bearing 4, a concave portion 5a having the same width in X-X direction(circular 6 direction) as the diameter of the bearing 4 and of the width in Y-Y direction(radial direction) larger than the maximum eccentricity of the bearing 4. The bearing 4 is fitted in the concave portion 5a of the rocking member 5.

Accordingly, when the second rotation shaft 3 rotates, the rocking member 5 reciprocally revolves round the axis 5b in X-X direction. The rocking member 5 is supported by supporting member 6, for example, as fitted in a hollow hole 6a thereof. The supporting member 6 is rotatably mounted in a fixing member 8 by means of a bearing 7.

In Fig. 1(a), 9 is a bearing fixing member, 10 is a washer, 11 is a fixing bolt and 12 is a sawing plate. The supporting member 6 has a hollow hole 6a with a thread 6b for threadedly engaging a fixing bolt 11. The sawing plate 12 can be secured at its end between the bearing fixing member 9 and the washer 10 by tightening the bolt 11. While the rotation shaft 1 rotates, the sawing plate 12 together with the supporting member 6 reciprocally revolve in the direction perpendicular to the section shown in Fig. 1(a).

The above-mentioned power transmission device, whereby rotational movement of a rotation shaft 1 is transmitted to a coaxially rotatable supporting member 6 to cause coaxial reciprocal revolution thereof, has to utilize an inclined rotation transmission mechanism 2 to make the supporting member 6 aligned with the rotation shaft 1, that not only complicates the construction of the device, but also requires the large-sized body case for accommodating the mechanism. This restricts the possibility of reducing the dimension of the device.

Therefore, it is an aim of the present invention to provide a power transmission device for converting rotational movement of a rotation shaft into reciprocal turning movement, comprising a first rotation shaft, a second rotation shaft rotatable with an eccentricity to the first rotation shaft, a bearing mounted on an end of the second rotation shaft, a rocking member swingable in response to only a circular displacement of the second rotation shaft round its revolving axis, and a turning shaft extending parallel to the first rotation shaft and fixing a swinging axis of the rocking member, characterized in that the device converts rotation of the first rotation shaft into reciprocal turning movement of the turning shaft.

According to the present invention, the power transmission device is constructed to convert rotational movement of the rotation shaft into reciprocal turning movement of a shaft extending parallel to the rotation shaft, thereby its mechanical construction is simplified with a reduced size of a body case for accommodating therein the turning shaft.

Fig. 1(a) is a sectional view of an essential portion of a power transmission device embodying the present invention. Fig. 1(b) is a view taken along line B-B of Fig. 1(a). In Figs. 1(a) and 1(b), parts similar in function to those shown in Figs. 2(a) and 2(b) are indicated with the same reference numerals.

The embodiment shown in Figs. 2(a) and 2(b) , rotational movement of a first rotation shaft 1 is transmitted to a turning shaft 15 without using a tilted rotation-transmitting mechanism 2 of the prior art shown in Fig. 1(a). In other words, the prior art device of Fig. 1(a) employs such a construction that a rotation shaft 1 and a supporting member 6 are disposed coaxially with each other to make a rocking member 5 rockably move round the supporting member 6, i.e., round a rotation axis of the rotation shaft 1 by using a tilted rotation shaft 2. Therefore, the body case 13 for holding a sawing plate 12 has a large diameter and is hard to handle. The device is expensive to manufacture because of its complicated mechanical construction.

On the contrary, the power transmission device according to the present invention is constructed in such a way that the rotation shaft 1 and the turning shaft 15 are arranged parallel to each other (i.e., with a step therebetween in the rocking member 5), thereby the rocking member 5 can be directly driven by the rotation shaft 1 without using the tilted rotation-transmitting shaft. The swinging axis of the rocking member 5 is connected with a bolt 5c or the like to the turning axis of the turning shaft 15, thereby the turning shaft 15 can reciprocally turn round its axis in the same manner as the supporting member of the prior art shown in Fig. 1(a).

A sawing plate 12 can be removably secured to a tip portion of the turning shaft 15 by loosening a bolt 11, then mounting the sawing plate 12 at the tip of the turning shaft 15 and then tightening the bolt 11 to secure it thereto.

The turning shaft 15 is rotatably supported by ball bearings 7 in the fixing portion (body case 13). Accordingly, when the rocking member 5 is driven by the rotation shaft 1, its movement is transmitted through the bolt 5c to the turning shaft 15 which in turn reciprocally turns to cause reciprocal movement of the sawing plate in the direction perpendicular to the section shown in Fig. 2(a).

As be apparent from the foregoing description, according to the present invention, it is possible to provide the power transmission device which is simple in construction, easy and reliable to operate and inexpensive to manufacture, since it employs the parallel arrangement of the rotation shaft 1 and the turning shaft 15, that eliminates the need of using the tilted transmitting mechanism and thereby allows the body case to have a reduced diameter for accommodating the turning shaft 15 only.

## Claims

1. A power transmission device for converting rotational movement of a rotation shaft into reciprocal turning movement, comprising a first rotation shaft, a second rotation shaft rotatable with an eccentricity to the first rotation shaft, a bearing mounted on an end of the second rotation shaft, a rocking member swingable in response to only a circular displacement of the second rotation shaft round its revolving axis, and a turning shaft extending parallel to the first rotation shaft and fixing a swinging axis of the rocking member, characterized in that the device converts rotational movement of the first rotation shaft into reciprocal revolving movement of the turning shaft.
